(19) Europäisches Patentamt
European Patent Office
Office européen des brevets

(11) **EP 2 180 905 B1**

(12) **EUROPEAN PATENT SPECIFICATION**

(45) Date of publication and mention
of the grant of the patent:
**07.09.2016 Bulletin 2016/36**

(21) Application number: **08773312.7**

(22) Date of filing: **10.07.2008**

(51) Int Cl.:
**A61L 15/44** *(2006.01)*       **A61K 9/70** *(2006.01)*

(86) International application number:
**PCT/DK2008/050176**

(87) International publication number:
**WO 2009/010068 (22.01.2009 Gazette 2009/04)**

(54) **A MEDICAL DRESSING COMPRISING AN ANTIMICROBIAL AGENT**

MEDIZINISCHE AUFLAGE MIT ANTIMIKROBIELLEM MITTEL

PANSEMENT MÉDICAL COMPRENANT UN AGENT ANTIMICROBIEN

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **13.07.2007 DK 200701037
13.07.2007 US 929820 P**

(43) Date of publication of application:
**05.05.2010 Bulletin 2010/18**

(73) Proprietor: **Coloplast A/S
3050 Humlebæk (DK)**

(72) Inventors:
• **LARSEN, Rasmus Dines
DK-2000 Frederiksberg (DK)**
• **REITZEL, Niels
DK-2840 Holte (DK)**

(56) References cited:
EP-A- 1 917 982          WO-A-02/062403
WO-A-2006/066752      WO-A-2007/068890
WO-A-2007/098772      GB-A- 2 428 581
US-A1- 2006 210 612

**Description**

**FIELD OF THE INVENTION**

This invention relates to medical dressings comprising absorbent polyurethane foam wherein the foam comprises two antimicrobial agents. This invention also relates to the use of such dressings.

**BACKGROUND OF THE INVENTION**

A wound, ulcer or sore may be defined as an injury to the skin and underlying tissue. Normally, a wound will heal by going through well-characterized healing phases, such as inflammation, granulation, epitheliazation and wound closure. All phases are regulated by a balanced action of proteases and signalling factors (cytokines and growth factors), which are essential to healing.

In a chronic wound, the healing phase has become trapped for reasons not entirely understood. A chronic wound is characterized by a prolonged inflammation, a failure to re-epithelialize and by defective remodelling of the extra-cellular matrix.

It is generally accepted that bacteria in a wound are detrimental for proper wound healing. Ulcers that are critically colonised with bacteria will often present themselves as painful, malodorous and with increased exudate levels. It is therefore of key importance to obtain and maintain low levels of bacteria and low virulence in the wound in order to avoid stalled wound healing and wound infection. The term critical colonisation has been applied to describe the stage in between normal contamination of a wound and overt clinical infection, and thus represents an unwanted clinical situation where bacterial imbalance is present in the ulcer.

Application of a dressing comprising and releasing an antimicrobial composition to a chronic wound, reduces the number of microbes, such as bacteria, by killing them. This will affect the inflammation by reducing it since the bacteria no longer trigger the body's reaction to the infectious agent. The protease concentration in chronic wounds is much higher than in acute wounds. The proteases originate from the body's own cells and from the bacteria, and when present in high concentrations, they degrade important ECM (extra cellular matrix) proteins as well as other essential proteins and growth factors. Thus, proteases in high amounts delay the wound healing. Antimicrobial agents and compositions present in a dressing will affect the wound healing positively and the patient's equality of life will increase.

A number of antimicrobial compositions and agents are described in the prior art. Silver based antimicrobial compositions are well known in this technical field.

WO 02/062403 and WO 02/078755 describe medical dressings comprising a complex comprising silver and a transitional element of group IV of the Periodic System of Elements, in particular the silver sodium hydrogen zirconium phosphate complex. These applications describe the preparation of polyurethane foam sheets and hydrogels comprising silver sodium hydrogen zirconium phosphate complex.

US 6093414 describes stable, purified silver-based antimicrobial compositions and processes for making such compositions, comprising carrier-free silver thiosulfate ion complexes either suspended in a base or incorporated into a matrix. These silver thiosulfate ion complex antimicrobial compositions are useful in the treatment and prevention of infections and diseases.

WO 2004/007595 describes flexible cellular polyurethane foam products. The foams are primarily comprised of: (a) a polyisocyanate component selected from the group consisting of toluenen diisocyanate (TDI), methylene diisocyanate (MDI), monomeric methylene diisocyanate (MDI), polymeric methylene diisocyanate (MDI), toluene diisocyanate (TDI) prepolymer, methylene diisocyanate (MDI) prepolymer, and combinations thereof; (b) an aqueous component including a polyol or polyol blend, or alternatively, a polyol or polyol blend component; and (c) a controlled release antimicrobial component such as silver and silver compounds. The resulting flexible cellular polyurethane foam products, containing the antimicrobial component, are suitable for use as wound dressings or other skin contact (e.g., medical and/or cosmetic) applications.

WO 2004/112805 describes an antimicrobial composition comprising silver and at least one compound, which interacts with a microbial cell wall to inhibit microbial silver resistance. The resistance inhibitors include molecules that can promote the transport of silver across the cell wall, and/or disrupt the cell wall to allow silver into the cell, and/or disrupt ion pump mechanisms in the cell wall for removing silver from the cell. Inhibitor compounds include fusaric acid, tocopherol, resveratrol, and myristic acid. Polyhexamethylene biguanide (PHMB) is also mentioned as a resistance inhibitor. The composition can be used in wound dressings.

Polyhexamethylene biguanide (PHMB or polihexanide) is a small cationic polymer with antimicrobial properties. It has a broad range of applications ranging from pool water cleaning agents to contact lens rinse solution preservation agents. Also it is suitable for establishing aseptic conditions in and around a healing wound.

WO 04/037115 discloses a medical dressing containing an antimicrobial agent. The medical dressing comprises a layered fabric, comprising an inner layer of substantially hydrophilic material, an outer layer of substantially hydrophobic material on both sides of the inner layer, and an antimicrobial agent. The antimicrobial agent may be releasably impregnated into the fabric, coated on said fabric or a combination thereof. The antimicrobial agent may be a biguanide such as poly-hexamethylene biguanide (PHMB). The fabric inner layer material may be substantially a cellulose fiber, preferably substantially rayon, and the fabric outer layer material may be substantially polyester, e.g., a combination of textile matrix grade polyester fiber and amorphous binder grade polyester fiber. The fabric is preferably treated with an aqueous solution of surfactant and PHMB to have about 1500-3500 ppm of extractable PHMB.

EP 0196459 discloses an adhesive surgical dressing with an antimicrobial agent in the adhesive. The antimicrobial agent is a salt of polyhexamethylene biguanide and is applied to the surface of the adhesive to a depth of not more than 50% of the thickness of the adhesive.

EP 0240097 describes a surgical dressing or incised drape material comprising a substrate coated with an antimicrobial containing adhesive. The antimicrobial is polyhexamethylene biguanide hydrochloride and is distributed in the adhesive as particles having a size from 20 to 300 microns.

Moist Wound Healing (MWH) dressings are capable of providing fast wound healing because they allow the surface of the wound to be kept moist while being capable of absorbing excessive wound fluid (exudate) to avoid degradation of the healing wound tissue and to prevent degradation of the surrounding skin (maceration). Such MWH dressings often employ hydrophilic materials such as hydrophilic foams as the wound contacting layer. Polyurethane foam is very suitable for providing a moist wound bed while removing excess amounts of exudate.

WO 2006/066752 describes microbicidal hydrophilic polyurethane foams that contain polyhexamethylene biguanide (PHMB) and/or the hydrochloride thereof and a super absorbent. The invention also relates to wound dressings produced thereof and to methods for producing the therapeutically active polyurethane foams and to the wound dressings produced thereof.

BR 9900032 relates to a sponge for general cleaning or for bath with a biocidal (bactericidal and fungicidal) agent incorporated into the polyurethane foam, characterized in that the polyurethane foam has, incorporated into it, biocides selected from those of the families polihexanide, benzisotiazolin, metallic pyridione, arsenic base, ammonium quaternary, at a concentration ranging from 0.1% to 3.0% within the product.

Now it has surprisingly been found that polyhexamethylene biguanide (PHMB) in a releasable form in a polyurethane foam not only shows the antimicrobial effect of PHMB but also benefits from an softening effect of PHMB on the polyurethane foam.

## SUMMARY OF THE INVENTION

This invention relates to medical dressings comprising absorbent polyurethane foam wherein the foam comprises a releasable form of poly-hexamethylene biguanide (PHMB) acting both as an anti-microbial agent as well as a softening agent and a silver ion complex with a transitional element of group IV of the periodic system of elements.. This invention also relates to the use of such dressings.

## BRIEF DESCRIPTION OF THE DRAWINGS

Figure 1 showing a diagram illustrating texture measurement of foam with PHMB (large hysteresis). The area under the graph A1= from anchor 1 -> 2 and A2 = from anchor 2 -> 5.

Figure 2 showing a diagram illustrating texture measurement of non-PHMB foam. The area under the graph A1= from anchor 1 -> 2 and A2 = from anchor 2 -> 5.

Figure 3 showing a diagram illustrating UV absorption at 236 nm (lambda max for PHMB) of Biatain AgP foam extract.

Figure 4 showing a diagram illustrating resilience of the foam (A2 / A1). The two 2nd order polynomial fits are given as a guide to the eye as to where the change in resilience starts to decrease.

Figure 5 showing a diagram illustrating the absorption under a pressure mimicking the absorption capacity of the dressing while being subjected to forces identical to those experienced under a compression bandage (i.e. under 40 mm Hg).

Figure 6 showing a diagram-illustrating zone of inhibition data of a polyurethane foam with silver and PHMB showing the added effect of the PHMB on inhibition zone sizes.

Figure 7 showing a test set up for analysis of absorption under pressure.

Figure 8 showing a diagram-illustrating plot of coefficients for resilience from MODDE 8 (Umetrics Inc.) statistical software for analysis of fractional factorial experiment design data.

## DETAILED DESCRIPTION OF THE INVENTION

The present invention combines moist wound healing principles and treatment of bacterial infection in wounds with improvement of the characteristics of the absorbent polyurethane foam during the healing process.

In one embodiment a medical dressing comprises absorbent polyurethane foam and an antimicrobial releasing agent comprised in the polyurethane foam wherein the antimicrobial releasing agent is polyhexamethylene biguanide (PHMB).

According to another embodiment of the invention the antimicrobial releasing agent is homogenously distributed in the polyurethane foam.

In order to perform well, the polyurethane foam intended to be used as absorbing material in wound care must be able to absorb wound exudates under a compression bandage (e.g. when used on leg ulcers). At the same time the dressing must be conformable. This is often in contradiction to being highly absorbent under pressure, since soft and conformable foam tends to get compressed and therefore cannot absorb properly.

It has now surprisingly been found that foam softness and conformability, which is also described as low resilience, can be controlled by adding the antimicrobial compound PHMB. A theory for the resilience controlling effect could be that the short chain length polymer interferes with the packing of the polyether-polyurethane cross-linked structure without actually forming covalent bonds to the polyether-polyurethane.

It is even more surprising that such conformable foam with reduced resilience still has a very high liquid absorption capacity (e.g. wound exudates) even under applied pressure and, furthermore, with the main part of the PHMB being releasable by dissolution in the wound exudate. This is indeed wanted in order to get the antimicrobial agent into the wound where it can exert its antimicrobial action.

The group of cationic small polymers/ small molecules consisting of Octenidine, Polihexanide and Taurolidine act by a cationic interaction mechanism targeting anionic residues on the surface of bacteria cell membranes and cell wall.

PHMB has a specificity for bacterial cell wall fatty acids but not for human cell wall more neutral fatty acids. A general property of antimicrobial polymers is that the activity of antimicrobial polymers is based on their special constitution, which comprises surface located functional groups and the three dimensional structure of the polymers. The antimicrobial efficacy is attributed only to the final polymer itself, not to leaching low molecular additives or the initial monomers.

Activity of PHMB increases on a weight basis with increasing levels of polymerization, which has been linked to enhanced inner membrane perturbation. Unlike chlorhexidine but similar to alexidine, PHMB causes domain formation of the acidic phospholipids of the cytoplasmic membrane. Permeability changes ensue, and there is believed to be an altered function of some membrane-associated enzymes.

The proposed sequence of events during its interaction with the cell envelope of E. coli is as follows:

(i) there is rapid attraction of PHMB toward the negatively charged bacterial cell surface with strong and specific adsorption to phosphate-containing compounds;
(ii) the integrity of the outer membrane is impaired, and PHMB is attracted to the inner membrane;
(iii) binding of PHMB to phospholipids occurs with an increase in inner membrane permeability (Potassium ion loss) accompanied by bacteriostasis;
(iv) complete loss of membrane function follows with precipitation of intracellular constituents and a bactericidal effect.

Antimicrobial means both antibacterial, antifungal and antiviral. Hence the term antimicrobial is the correct description for both silver ions and PHMB, however, within the field of wound care, where infection is mostly related to bacteria, the term antibacterial is sometimes used.

The antimicrobial releasing agent of PHMB may be incorporated into the polyurethane foam together with other antimicrobial and/or antibacterial agents such as silver containing compounds in order to obtain a broader and stronger antimicrobial effect of the dressing.

According to one embodiment of the invention a medical dressing comprises absorbent polyurethane foam, said polyurethane foam comprising two antimicrobial releasing agents, wherein the first antimicrobial releasing agent is polyhexamethylene biguanide (PHMB) and the second antimicrobial releasing agent is complex of silver ions with a transitional element of group IV of the periodic system of elements.

PHMB is highly active against gram(+) bacteria and hence is the ideal supplement to the present state of the art antimicrobial dressings, which are silver containing.

The present invention can be carried out with various types of foam material. The types of foam material and their manufacture are known to the skilled person. Preferred foamed materials are hydrophilic polyether based polyurethane.

Low resilience foam is also termed "slow recovering", "memory" and "viscoelastic". Foam having such a property is characterized by leaving a temporary imprint in the foam after compression. Such foam provides a superior comfort to the patient by spreading the load and reducing pressure hence reducing the risk for trauma on the often very fragile skin surrounding ulcers.

In one embodiment of the invention both the antimicrobial releasing agent of PHMB and the antimicrobial agent comprising silver ions is contained in one single absorbent element capable of absorbing wound exudates. Typically, the absorbent element is a layer of polyurethane foam containing a mixture of the anti-microbial releasing agent of PHMB and the antimicrobial agent comprising silver ions, the compounds being homogenously distributed in the polyurethane foam.

The antimicrobial releasing agent of PHMB and the antimicrobial agent comprising silver ions may also be contained in two separate absorbing elements capable of absorbing wound exudates. As an example, the dressing comprises two layers of polyurethane foam where one foam layer comprises the antimicrobial releasing agent of PHMB homogenously distributed therein and the other foam layer comprises the antimicrobial agent comprising silver homogenously distributed therein.

According to an alternative embodiment of the invention, the dressing comprises a first and a second absorbent element capable of absorbing wound exudates, said first and second absorbent elements comprising an antimicrobial releasing agent of PHMB and an antimicrobial agent comprising silver ions, where the amount of antimicrobial releasing agent of PHMB is higher in said first absorbent element than in said second absorbent element, and the amount of an antimicrobial agent comprising silver ions is higher in said second absorbent element than in said first absorbent element. Typically, a dressing according to this embodiment of the invention comprises two layers of polyurethane foam on top of each other.

In yet another embodiment of the invention the dressing comprises a first and a second absorbent element where the first element is a centre circular element with a diameter from 5 to 10 cm, and the second element a ring encircling the first element. Each element may comprise one or both of the pharmaceutically active agents. In one embodiment the antimicrobial compound comprising silver is contained in the absorbent centre circular element and the antimicrobial releasing agent of PHMB is contained in the absorbent ring element surrounding the centre circular element.

The absorbent elements capable of absorbing wound fluid may in principle be placed anywhere in the dressing.

Preferably, the absorbent element capable of absorbing wound exudates is in the form of one layer, or two layers on top of each other.

Preferably, the absorbent element capable of absorbing wound exudates is a foam, most preferred a polyurethane foam, capable of absorbing wound exudates.

Being absorbent is defined by the ability of absorbing liquids, suspensions, moisture, exudate, body fluid, pus or the like under a pressure load equivalent to 40 mmHg as described in the experimental part. A material is defined as absorbent when it is capable of absorbing at least 0.1 times its own weight.

The foam dressing of the present invention is preferably capable of absorbing 0.1 - 60 times its own weight under pressure of 40 mmHg, more preferred 0.5 - 40 times its own weight, even more preferred 5 - 20 times its own weight and most preferred 8 - 15 times its own weight.

In one embodiment of the invention the absorption capacity under a pressure load (as defined in the experimental part) is higher than 5 g water/g dry foam.

The foam dressing of the present invention is preferably containing 0.01 - 14 % (w/w) of PHMB in the dry polyurethane foam matrix, more preferred 0.05 - 6.8% (w/w) of PHMB in the dry polyurethane foam matrix, and most preferably between 0.1 and 6.8% (w/w) of PHMB in dry polyurethane foam matrix in a releasable form.

In one embodiment of the invention the concentration of the releasable amount of PHMB is between 0.006% and 10.3% (w/w), more preferably between 0.03% and 4.1% (w/w) and most preferably between 0.06 and 4.1% (w/w) in dry foam.

The foam dressing of the present invention is preferably having a resilience (as defined below) between 0.1 and 0.9, more preferred between 0.25 - 0.85 and most preferred between 0.35 and 0.8.

According to one preferred embodiment of the invention, the dressing consists of one foam layer, or two foam layers on top of each other.

A skin friendly adhesive may be present on the wound contact surface of the dressing according to the invention. In one embodiment the adhesive is present directly on the surface of the foam layer(s) or around the absorbent element, see e.g. US 2005113733 and WO 99/61077.

A release liner may protect the adhesive surface.

A dressing of the invention comprising two foam layers with different content of therapeutically active ingredients may comprise a removable top film on both sides of the dressing. Before use the removable top film is removed from the surface of the dressing, which is to be placed towards the wound surface.

The antimicrobial agent comprising silver ions may be selected from silver sulphadiazine, silver nitrate, silver acetate, silver lactate, silver sulphate, silver sodium thiosulphate, or silver chloride, and zeolites comprising silver.

The dressing comprises a complex of silver ions with a transitional element of group IV of the periodic system of elements.

According to an embodiment of the invention, the complex of silver ion and a transitional element of group IV of the periodic system of elements is homogenously distributed in the polyurethane foam.

By a transitional element of group IV of the periodic system of elements is meant titanium, zirconium or hafnium. The complex of silver ions and a transitional element of group IV of the periodic system of elements is preferably a silver sodium hydrogen zirconium phosphate complex.

Silver sodium hydrogen zirconium phosphate complex is marketed by Milliken under the trademark Alphasan. Alphasan is available in three grades: Alphasan

RC 2000 (comprising 10% silver ion), Alphasan 5000 (comprising 3.8% silver ion) and Alphasan RC 7000 (comprising 3.1% silver ion and 69% zinc oxide).

The amount of the complex of silver ion and the transitional element of group IV of the periodic system of elements in the dressing is typically in the range from 0.01 to 30 mg silver ion/cm$^2$ dressing when calculated as the amount of silver ions, more preferred silver ions is 0.1 - 8 mg/cm$^2$ dressing, and even more preferred 0.5 - 1.75 mg/cm$^2$ dressing.

As explained above it has now surprisingly been found that foam softness and conformability, which is also described as low resilience, can be controlled by adding the antimicrobial compound PHMB. Furthermore, it has now surprisingly been found that PHMB can be used as a general agent for decreasing the resilience of the foam when other agents, chemical powders or compounds are added to the foam and thereby increasing the resilience. The addition of silver increases the resilience, however the foam with silver and PHMB benefit from the decreasing effect of PHMB.

The antimicrobial releasing agent of PHMB and the additional added antimicrobial agent(s) are comprised in the absorbent element(s) so that wound exudates are easily brought into contact with the antimicrobial releasing agent of PHMB and the additional added antimicrobial agent(s) thereby allowing release to the wound.

Such an absorbing element or combination of absorbing elements comprising the active substances may in one embodiment constitute a dressing of the invention.

The absorbing element can be secured to the desired site using conventional means such as a cover dressing.

The dressing of the invention has mainly been described with reference to wound dressings but it will be evident for the skilled in the art that the invention is not limited to wound dressings. Thus, a medical dressing of the invention may be in the form of a wound dressing or an ostomy appliance or a dressing for covering an incision site in the skin.

According to one embodiment of the invention, the medical dressing is a wound dressing.

The dressing of the invention may comprise a skin-contacting surface comprising an area showing a skin friendly adhesive.

Such a dressing may also comprise a substantially water-impervious layer or film on the surface of the dressing facing away from the wound.

In one embodiment of the invention the dressing comprises a water impervious but vapour permeable backing layer

The adhesive may be any skin-friendly adhesive known per se, e.g. an adhesive comprising hydrocolloids or other moisture absorbing constituents, such as the adhesives disclosed in US patent No. 4,231,369 and in US patent No. 4,367,732, comprising hydrocolloids. Other types of skin-friendly adhesives may be silicone adhesives or polyurethane adhesives known per se.

A water impervious layer or film may be of any suitable material known per se for use in the preparation of dressings e.g. a polyurethane, polyethylene, polyester or polyamide film. A suitable material for use as a water impervious film is a polyurethane such as the low friction film material disclosed in US patent No. 5,643,187.

According to one embodiment of the invention the medical dressing is used for moisture absorption.

## EXPERIMENTAL PART

The following experimental techniques were used:

### Foam formulation

The foam was made using standard lab equipment: An aqueous phase (distilled water with between 0.05% - 5% w/w surfactant and the appropriate concentration of Cosmocil CQ (PHMB solution) was weighed into a single use plastic cup. Other ingredients, such as a silver complex, can be mixed into the water phase while stirring. Isocyanate end capped prepolymers were weighed into a different cup (in hood). The water phase was stirred once more just before being added to the pre-polymer phase, and the two phases were mixed with a propeller for 15 seconds and quickly poured onto a siliconized paper. Another siliconized paper is put on top, and the mixture is rolled, guided to a certain thickness by two distance pieces. The foam was allowed to rest in the hood for 3 minutes, after which the foam pieces were dried in an oven at 80 deg C for 45 min and chilled at room temperature.

**Resilience measurement**

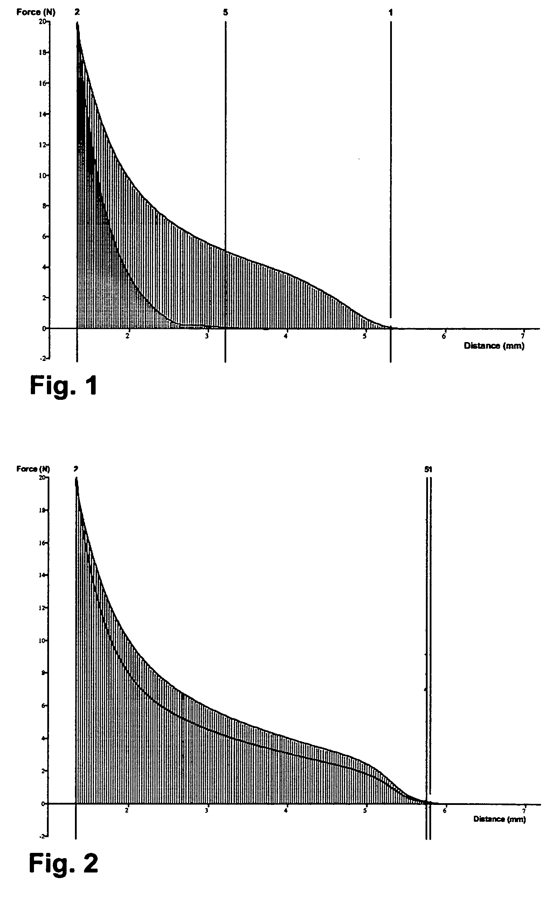

The resilience measurement was made at 23 deg C / 50% RH with a Texture Analyser TA.XT.Plus (Stable micro systems) using a Ø=20 mm cylindrical probe. The flat bottom side of the cylindrical probe was indented into the foam until the force exerted by the foam reaches 20 N, then the probe was subtracted from the foam. The speed of the probe was constant during a measurement cycle. A test speed of 0.5 mm/sec was used during the measurements. During the entire measurement, the force exerted by the foam to the probe was recorded, and a distance - force curve - was plotted as seen in Figures 1 and 2. The figures illustrate Distance in mm at the x-axis and Force in N at the y-axis. The area under the compression curve is called A1 and the area under the retraction curve is called A2. A1 can be envisaged as the foam hardness, and A2 is one measure of the resilience. Also the ratio A2/A1 is called the normalized resilience (since it is normalized to the area under the compression curve A1).

Equipment

    Test tubes of glass with screw cap 10 ml: Schott GL18
    Perkin Elmer Lambda 25 UV-VIS spectrophotometer
    1 cm quartz cuvettes
    For calibration: 20% PHMB solution (for instance Cosmocil CQ)

Calibration curve (standard solutions)

If necessary, a calibration curve can be made by making the following standard solutions and testing by UV spectroscopy as described below.

First a stock solution was made by diluting 1 mL Cosmocil CQ to 100 mL with deionized water. From this stock solution 0.5 mL was taken into each of the following measuring flasks and diluted with deionized water: 50 mL, 100 mL, 250 mL, 500 mL and 1000 mL.

Sample preparation

A foam sample was punched out in 2 mm x 2 mm pieces and between 0.27 g and 0.33 g (weighed with mg. accuracy) of such foam pieces was put in a sealed test tube (15 mL) and 10.00 mL deionized water was added. The tubes were shaken with 1000 rpm. for at least 1½ hours and max. 24 hours. 1.00 mL of this extract was transferred with a pipette to a 100 mL measuring beaker and filled with deionized water.

UV-Spectroscopy

Cuvettes were filled with extract or standard solution. One cuvette was filled with deionized water and was used to autozero.
The UV spectrum (200 nm to 400 nm) was measured (auto zeroed against water) with a scanning speed of 240 nm/min. By using the software Spectrum ver 5.0, the peak height at 236 nm was measured with an appropriate background subtraction. A background could for instance be a linear background between 220 nm and 260 nm. The peak height at 236 nm was subsequently converted into a PHMB concentration by application of the calibration curve described above.

Zone of inhibition

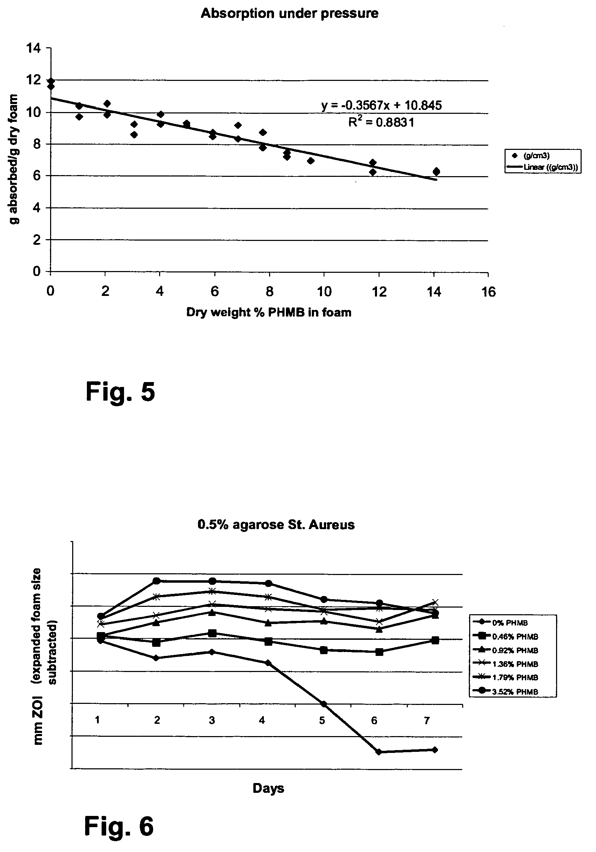

ZOI describes the clear zones that form if the product inhibits bacterial growth. Suspensions with app. 105 CFU/ml were plated on Iso Sensitest agar plates. Product samples of Ø10 mm were placed in triplets on the challenged agar plates and the plates were incubated at 37°C for 24 hours, where after the ZOI was measured and recorded (Figure 5). The test products were then transferred to new agar plates with fresh challenge bacteria and incubated another 24 hours and the ZOI was measured again. This procedure was repeated for 7 days.

**Absorption under pressure**

The test was used for analysis of foam bandages under pressure of 40 mmHg. The test simulated the use of a foam bandage under compression. The setup was made so maximal stability was obtained by keeping the center of mass beneath the actual sample.

App aratus/Chemicals

Petri dishes, (Ø140mm)
Filter plate in glass (Euro-Glass, por 00, Ø60mm)
An analytical balance (0.0001 g)
Punch mould (030 mm)
Solution A (8.298 g NaCl + 0.368 g CaCl$_2$, 2H$_2$O pr. Liter)
Weight on a hanger
White POM plate (Ø60mm)

The total weight of weight, hanger and POM plate was 386 g, corresponding to a 40mmHg pressure on a 030 sample.

Method

The analysis was made at room temperature.

Punch the sample (Ø30mm).
Measure thickness ([THICKNESS] in mm).
Weight the sample ([WIEGHT DRY] in g).
Put the filter plate (060 mm) into a petri dish (0140 mm).
Place the filter plate with the ragged side down.
Place the sample between the filter plate and POM plate (060).

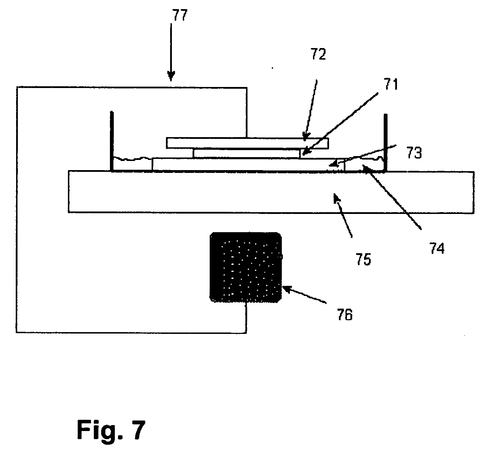

On the top of POM plate a hanger with a weight is placed. The test setup is illustrated in Figure 7 explained by the following reference numbers:

(71) Product/Sample (030 mm)
(72) White POM plate (Ø60mm)
(73) Filter plate (Ø60mm)
(74) Solution A
(75) Table
(76) Weight
(77) Stainless steel (Ø4mm)

It is very important that the sample is in the center, the hanger is vertical and the weight is "free hanging". 45 mL Solution A mixes carefully with the petri dish.

Wait 90 minutes *.
Remove solution A from the petri dish.
Remove the weight.
Weight the sample ([WEIGHT WET] in g).

* Results are time dependent. There is a tendency of lower results (5-10%) if you wait 24 hours. It is noted if the analysis time has been changed.

Results

Analyze data: The test thickness [THICKNESS] in mm, the test weight before the test [WEIGHT DRY] in g, test weight after test [WEIGHT WET] in g and [WEIGHT PRESS] was the test weight after role.

The samples' absorptions and retention was calculated as follows:

$$\text{Absorption under pressure} \left(\frac{g}{g}\right) = \left(\frac{[\text{WEIGHT WET}] - [\text{WEIGHT DRY}]}{[\text{WEIGHT DRY}]}\right)$$

## EXPERIMENTAL RESULTS

Acceptable range of PHMB

A large range of PHMB was tested in a hydrophilic polyurethane dressing. The PHMB range tested results in a PHMB concentration of up to 14% w/w in dry foam. It was indeed possible to incorporate such a high amount of PHMB into the foam without the foam collapsing.

Release of PHMB from foam

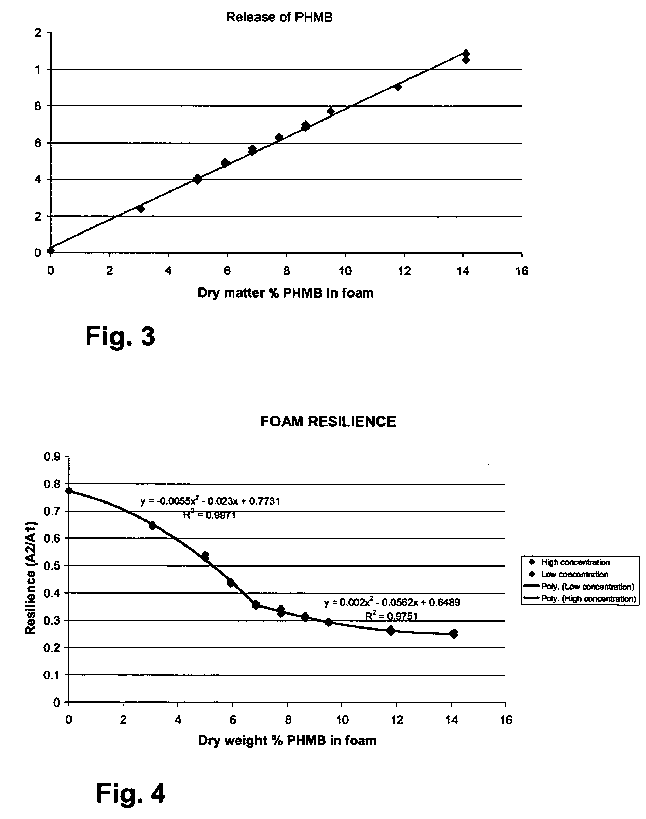

In Figure 3 it is seen that there is a linear release (by aqueous extraction as described in the experimental part) from the foam into an aqueous phase, showing that the PHMB amount released from the foam is a linear function of the concentration of the PHMB concentration in the foam. The figure illustrates Dry matter % PHMB in a foam at the x-axis and ABS at 236 nm of extract at the y-axis.
By application of a calibration curve as described previously, conversion of the ABS at 236 nm values into concentration of PHMB results in an amount of releasable up to 10.3% w/w, i.e. the weight of dry PHMB extracted from dry foam.

Foam resilience

The decrease in the resilience level is shown in Figure 4. The figure illustrates Dry weight % PHMB in a foam at the x-axis and Resilience (A2/A1) at the y-axis. Within the concentration range of PHMB shown in Figure 4, there is approx. a 3 fold decrease in the resilience (from 0.78 at 0% PHMB to 0.25 at 14% PHMB). The resilience decrease is shown to break around 6.8 % w/w PHMB in the foam as shown in Figure 4 as the two polynomial fit (that are only shown as a guide to the eye).

Absorption under pressure

As the PHMB content of the polyurethane foam is increased, the absorption under pressure decreases as shown in Figure 5. However even at 14 % of PHMB the absorption capacity of solution A into the foam is high (above 5 g / g dry foam). The figure illustrates Dry weight % PHMB in foam at the x-axis and g absorbed/g dry foam at the y-axis.

7 days dynamic Zone of Inhibition (0.5% agarose St. Aureus)

Figure 6 shows the antibacterial efficacy (against St. Aureus) of a silver containing foam with varying amounts of PHMB tested for 7 days. The figure illustrates Days at the x-axis and mm ZOI (expanded foam size subtracted) at the y-axis. It is seen that increasing the amount of PHMB increases the size of the inhibition zones. It is also seen that the addition of PHMB ensures 7 days of inhibition against St. Aureus.

**Conclusion on the experiments mentioned above**

In the experiments above it is demonstrated that by adding PHMB to the foam it is indeed possible to use PHMB as a means to control the foam resilience (down to A2/A1 = 0.25) resulting in a foam having a linear release of PHMB (see Figure 3) which ensures a good antibacterial efficiency as shown in Figure 6.

PHMB used as a general agent to decrease resilience

PHMB used as general agent to decrease polyurethane foam resilience with addition of other substances. The experiments presented in this example were made in order to substantiate the findings regarding work on incorporating poly-hexanide (PHMB) in polyurethane a foam dressing and using PHMB as a general agent for decreasing the resilience of the foam including when other agents, chemicals powders or compounds were added to the foam and thereby increasing the resilience.

In the laboratory report INF236 PHMB was tested in a polyurethane foam dressing in addition with Alphasan silver complex powder and/or titanium dioxide powder. The Alphasan, titanium dioxide, PHMB range tested was varied in a designed experiment using the MODDE software (Umetrics Inc) to statistically investigate interactions and effects of each component upon foam resilience (A2/A1). The range of the three additives was from 0 to 10 w/w % and the experiment was designed as a screening experiment, with fractional factorial design (Res. III) with pseudo resolution level 3. It was indeed possible to incorporate such a high amount of Alphasan powder and/or titanium dioxide powder and/or PHMB into the foam without collapsing the foam. The foams could be used as wound dressings, however the foams with lower resilience were considered more suitable for such use due to the better conformability of the foam to the patients wound surface.

Method

The foam was made using standard lab equipment: An aqueous phase (distilled water with between 0.05% - 5% w/w surfactant) and the appropriate concentration of Cosmocil CQ (PHMB solution) was weighed into a single use plastic cup. Other ingredients, such as a silver complex and/or titanium dioxide may then be mixed into the water phase while stirring. Concentrations of individual test runs were listed in Table 1. Isocyanate end capped prepolymers were weighed into a different cup (in hood). The water phase was stirred once more just before being added to the prepolymer phase, and the two phases were mixed with a propeller for 15 seconds and quickly poured onto a siliconised paper. Another siliconised paper was put on top, and the mixture was rolled, guided to a certain thickness by two distance pieces. The foam was allowed to rest in the hood for 3 minutes, after which the foam pieces were dried in an oven at 60 deg C for 60 min to complete dryness and chilled at room temperature. The resilience of the foams were measured as described above.

Statistical analysis and fit of model

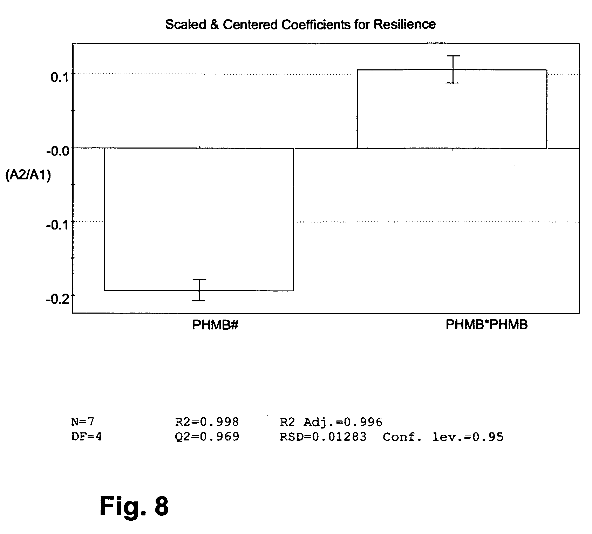

The results were entered into MODDE software (Umetrics Inc) in order to statistically investigate interactions and effects of each component upon foam resilience (A2/A1). The results are listed in Table 1 and the statistical analysis and fit of a statistical model are described in Figure 8 and Table 2. In the MODDE software a statistical model was fitted and thereby identified the significantly contributing factors, being mainly PHMB and PHMB*PHMB, as described in Figure 8 and Table 2. The inclusion of Alphasan silver complex and/or Titanium dioxide powder in the statistical fitting procedure only resulted in insufficient fit of the model having a low Q square ($Q^2$) (coefficient of model cross validation) without contributing with a statistically significant effect, i.e. not affecting the resilience of the foam. In general, a Q square similar in value to a R square indicates a good statistical fit to the model. Hence the correct statistical model fit only includes PHMB and PHMB*PHMB. A negative value of a first order coefficient describes that the factor is contributing to lower the resilience.

Conclusion

The results presented in Table 1 and Figure 8 and Table 2 clearly show that addition of PHMB to polyurethane foam decreases the resilience (A2/A1) of the foam structure even when adding high concentrations of Alphasan silver complex and/or titanium dioxide powder in the foaming process. This clearly indicates that PHMB is a general agent to decreasing the resilience of polyurethane foams.

Table 1. Experiment design table and results of the measured resilience of the foams. The resilience is lower for the samples with PHMB than for the samples without PHMB.

| Exp No | Exp Name | Run Order | Alphasan Silver Complex | TiO2 | PHMB | Resilience A2/A1 |
|--------|----------|-----------|-------------------------|------|------|------------------|
|        |          |           | % w/w                   | %w/w | % w/w |                 |
| 1 | No1 | 6 | 0 | 0 | 10 | 0.2452 |
| 2 | No2 | 2 | 10 | 0 | 0 | 0.7227 |
| 3 | No3 | 5 | 0 | 10 | 0 | 0.6959 |
| 4 | No4 | 4 | 10 | 10 | 10 | 0.2268 |
| 5 | No5 | 3 | 5 | 5 | 5 | 0.3204 |
| 6 | No6 | 7 | 5 | 5 | 5 | 0.3044 |

(continued)

| Exp No | Exp Name | Run Order | Alphasan Silver Complex | TiO2 | PHMB | Resilience A2/A1 |
|--------|----------|-----------|--------------------------|------|------|-------------------|
| 7 | No7 | 1 | 5 | 5 | 5 | 0.3142 |

Table 2. Summary of coefficients for resilience with 95% confidence intervals for each parameter from MODDE 8 (Umetrics Inc.) statistical software for analysis of fractional factorial experiment design data.

| | Scaled & Centered Coefficients for Resilience | 95% Confidence interval |
|---|---|---|
| Constant | 0.313 | 0.0205683 |
| PHMB | -0.193224 | 0.014544 |
| PHMB*PHMB | 0.106433 | 0.0181395 |
| Alphasan | no statistical significant contribution (excl from fit) | |
| Alphasan*Alphasan | no statistical significant contribution (excl from fit) | |
| TiO2 | no statistical significant contribution (excl from fit) | |
| TiO2*TiO2 | no statistical significant contribution (excl from fit) | |

**Claims**

1.  A medical dressing comprising absorbent polyurethane foam, said polyurethane foam comprising two antimicrobial releasing agents, wherein the first antimicrobial releasing agent is polyhexamethylene biguanide (PHMB) and the second antimicrobial releasing agent is complex of silver ions with a transitional element of group IV of the periodic system of elements.

2.  The medical dressing according to claim 1, wherein the complex of silver ion and a transitional element of group IV of the periodic system of elements are homogenously distributed in the polyurethane foam.

3.  The medical dressing according to any of the preceding claims, wherein the complex of silver ion and a transitional element of group IV of the periodic system of elements are silver sodium hydrogen zirconium phosphate complex.

4.  The medical dressing according to any of the preceding claims, wherein the amount of the complex of silver ion and a transitional element of group IV of the periodic system of elements correspond to a silver ion amount of 0.01-30 mg silver/cm$^2$ in the dressing.

5.  The medical dressing according to any of the preceding claims, wherein the anti-microbial releasing agent of PHMB is homogenously distributed in the polyurethane foam.

6.  The medical dressing according to any of the preceding claims, wherein the concentration of PHMB is between 0.01% and 14% (w/w), more preferably between 0.05% and 6.8% (w/w) and most preferably between 0.1 and 6.8% (w/w) in dry foam.

7.  The medical dressing according to any of the preceding claims, wherein the concentration of the releasable amount of PHMB is between 0.006% and 10.3% (w/w), more preferably between 0.03% and 4.1% (w/w) and most preferably between 0.06 and 4.1% (w/w) in dry foam.

8.  The medical dressing according to any of the preceding claims, wherein the polyurethane foam comprises hydrophilic polyether based polyurethane.

9.  The medical dressing according to any of the preceding claims, wherein the resilience of the foam, A2/A1 (as defined in the experimental part), is between 0.1 and 0.9, more preferred 0.25 - 0.85 and most preferred between 0.35 and 0.8.

10. The medical dressing according to any of the preceding claims, wherein the absorption capacity under a pressure load (as defined in the experimental part) is higher than 5 g water/g dry foam.

11. The medical dressing according to any of the preceding claims, wherein the dressing comprises a water impervious but vapour permeable backing layer

12. The medical dressing according to any of the preceding claims, wherein the dressing is a wound dressing.

**Patentansprüche**

1. Medizinisches Verbandmaterial, umfassend absorbierenden Polyurethanschaumstoff, wobei der Polyurethanschaumstoff zwei Antimikrobiotikumfreisetzende Mittel umfasst, wobei das erste Antimikrobiotikum-freisetzende Mittel Polyhexamethylenbiguanid (PHMB) ist und das zweite Antimikrobiotikum-freisetzende Mittel ein Komplex von Silberionen mit einem Übergangselement der Gruppe IV des Periodensystems der Elemente ist.

2. Medizinisches Verbandmaterial gemäß Anspruch 1, wobei der Komplex von Silberionen mit einem Übergangselement der Gruppe IV des Periodensystems der Elemente homogen in dem Polyurethanschaumstoff verteilt ist.

3. Medizinisches Verbandmaterial gemäß einem der vorstehenden Ansprüche, wobei der Komplex von Silberionen mit einem Übergangselement der Gruppe IV des Periodensystems der Elemente ein Silber-Natriumhydrogen-Zirconium-Phosphat-Komplex ist.

4. Medizinisches Verbandmaterial gemäß einem der vorstehenden Ansprüche, wobei die Menge des Komplexes von Silberionen mit einem Übergangselement der Gruppe IV des Periodensystems der Elemente einer Silberionen-Menge von 0,01-30 mg Silber/cm$^2$ in dem Verbandmaterial entspricht.

5. Medizinisches Verbandmaterial gemäß einem der vorstehenden Ansprüche, wobei das Antimikrobiotikum-freisetzende Mittel von PHMB homogen in dem Polyurethanschaumstoff verteilt ist.

6. Medizinisches Verbandmaterial gemäß einem der vorstehenden Ansprüche, wobei die Konzentration von PHMB zwischen 0,01 % und 14 % (w/w), bevorzugter zwischen 0,05% und 6,8% (w/w) und höchst bevorzugt zwischen 0,1 und 6,8 % (w/w) in trockenem Schaumstoff beträgt.

7. Medizinisches Verbandmaterial gemäß einem der vorstehenden Ansprüche, wobei die Konzentration der freisetzbaren Menge von PHMB zwischen 0,006 % und 10,3 % (w/w), bevorzugter zwischen 0,03% und 4,1 % (w/w) und höchst bevorzugt zwischen 0,06 und 4,1 % (w/w) in trockenem Schaumstoff beträgt.

8. Medizinisches Verbandmaterial gemäß einem der vorstehenden Ansprüche, wobei der Polyurethanschaumstoff hydrophiles Polyurethan auf Polyetherbasis umfasst.

9. Medizinisches Verbandmaterial gemäß einem der vorstehenden Ansprüche, wobei die Nachgiebigkeit des Schaumstoffs A2/A1 (wie im Experimentalteil definiert) zwischen 0,1 und 0,9 beträgt, bevorzugter 0,25-0,85 und höchst bevorzugt zwischen 0,35 und 0,8.

10. Medizinisches Verbandmaterial gemäß einem der vorstehenden Ansprüche, wobei die Absorptionskapazität unter einer Drucklast (wie im Experimentalteil definiert) höher als 5 g Wasser/g an trockenem Schaumstoff ist.

11. Medizinisches Verbandmaterial gemäß einem der vorstehenden Ansprüche, wobei das Verbandmaterial eine wasserundurchlässige aber dampfdurchlässige Trägerschicht umfasst.

12. Medizinisches Verbandmaterial gemäß einem der vorstehenden Ansprüche, wobei das Verbandmaterial ein Wundverbandmaterial ist.

**Revendications**

1. Pansement médical comprenant une mousse de polyuréthane absorbante, ladite mousse de polyuréthane comprenant deux agents de libération antimicrobiens, dans lequel le premier agent de libération antimicrobien est le polyhexaméthylène-biguanide (PHMB) et le deuxième agent de libération antimicrobien est un complexe d'ions d'argent avec un élément de transition du groupe IV du système périodique des éléments.

2. Pansement médical selon la revendication 1, dans lequel le complexe d'ion d'argent et d'un élément de transition du groupe IV du système périodique des éléments est distribué de façon homogène dans la mousse de polyuréthane.

3. Pansement médical selon l'une quelconque des revendications précédentes, dans lequel le complexe d'ion d'argent et d'un élément de transition du groupe IV du système périodique des éléments est un complexe argent-hydrogénophosphate de zirconium-sodium.

4. Pansement médical selon l'une quelconque des revendications précédentes, dans lequel la quantité du complexe d'ion d'argent et d'un élément de transition du groupe IV du système périodique des éléments correspond à une quantité d'ion d'argent de 0,01 à 30 mg d'argent/cm$^2$ dans le pansement.

5. Pansement médical selon l'une quelconque des revendications précédentes, dans lequel l'agent de libération antimicrobien PHMB est distribué de façon homogène dans la mousse de polyuréthane.

6. Pansement médical selon l'une quelconque des revendications précédentes, dans lequel la concentration de PHMB est comprise entre 0,01 % et 14 % (m/m), plus préférablement entre 0,05% et 6,8% (m/m) et de manière préférée entre toutes entre 0,1 et 6,8 % (m/m) dans la mousse sèche.

7. Pansement médical selon l'une quelconque des revendications précédentes, dans lequel la concentration de la quantité libérable de PHMB est comprise entre 0,006 % et 10,3 % (m/m), plus préférablement entre 0,03% et 4,1% (m/m) et de manière préférée entre toutes entre 0,06 et 4,1 % (m/m) dans la mousse sèche.

8. Pansement médical selon l'une quelconque des revendications précédentes, dans lequel la mousse de polyuréthane comprend un polyuréthane à base de polyéther hydrophile.

9. Pansement médical selon l'une quelconque des revendications précédentes, dans lequel l'élasticité de la mousse, A2/A1 (telle que définie dans la partie expérimentale), est comprise entre 0,1 et 0,9, plus préférablement 0,25 et 0,85 et de manière préférée entre toutes entre 0,35 et 0,8.

10. Pansement médical selon l'une quelconque des revendications précédentes, dans lequel la capacité d'absorption sous une charge de pression (telle que définie dans la partie expérimentale) est supérieure à 5 g d'eau/g de mousse sèche.

11. Pansement médical selon l'une quelconque des revendications précédentes, **caractérisé en ce que** le pansement comprend une couche de support imperméable à l'eau mais perméable à la vapeur.

12. Pansement médical selon l'une quelconque des revendications précédentes, **caractérisé en ce que** le pansement est un pansement de plaie.

Fig. 1

Fig. 2

Fig. 3

Fig. 4

**Absorption under pressure**

$$y = -0.3567x + 10.845$$
$$R^2 = 0.8831$$

Legend:
- ♦ (g/cm3)
- —— Linear ((g/cm3))

X-axis: Dry weight % PHMB in foam
Y-axis: g absorbed/g dry foam

## Fig. 5

**0.5% agarose St. Aureus**

Legend:
- 0% PHMB
- 0.46% PHMB
- 0.92% PHMB
- 1.36% PHMB
- 1.79% PHMB
- 3.52% PHMB

X-axis: Days
Y-axis: mm ZOI (expanded foam size subtracted)

## Fig. 6

**Fig. 7**

Scaled & Centered Coefficients for Resilience

N=7  R2=0.998 R2 Adj.=0.996
DF=4  Q2=0.969 RSD=0.01283 Conf. lev.=0.95

# Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02062403 A
- WO 02078755 A
- US 6093414 A
- WO 2004007595 A
- WO 2004112805 A
- WO 04037115 A
- EP 0196459 A
- EP 0240097 A

- WO 2006066752 A
- BR 9900032
- US 2005113733 A
- WO 9961077 A
- US 4231369 A
- US 4367732 A
- US 5643187 A